# EUROPEAN PATENT SPECIFICATION

(11) **EP 0 793 489 B1**
(45) Date of publication and mention of the grant of the patent: **29.03.2006**
(21) Application number: 95934980.4
(22) Date of filing: 13.09.1995
(51) Int. Cl.: A61K 31/35, A61P 17/00, A61P 17/10

(54) **USE OF STATIN FOR THE TREATMENT OF SKIN DISORDERS**
VERWENDUNG VON STATIN ZUR BEHANDLUNG VON HAUTERKRANKUNGEN
UTILISATION DE STATIN POUR LE TRAITEMENT DES MALADIES DE LA PEAU

(30) Priority: 13.09.1994 IL 11094394
(43) Date of publication of application: 10.09.1997
(73) Proprietor: Ramot at Tel-Aviv University Ltd., Tel Aviv 69975 (IL)
(72) Inventor: SAVION, Naphtali, 54052 Givat Shmuel (IL); BRENNER, Sara, Herzlia-Pituach (IL)
(74) Representative: Vossius & Partner
(86) International application number: PCT/US1995/011678
(87) International publication number: WO 1996/008248

(56) References cited:
- EP-A- 0 596 326
- EP-A- 0 599 203
- WO-A-87/04346
- WO-A-92/00076
- WO-A-94/21230
- GB-A- 1 026 927
- GB-A- 2 081 580
- US-A- 3 206 460
- US-A- 4 231 938
- US-A- 5 075 327

## Description

### FIELD OF THE INVENTION

The present invention is generally in the field of compositions for topical application onto the skin intended to improve the skin's condition. The present invention provides compositions useful for improving various skin conditions, in particular acne.

### BACKGROUND OF THE INVENTION

Acne is a chronic inflammatory disorder of the pilosebaceous follicles, particularly in the face and neck region, occurring most commonly in adolescence between the ages of about 14 to about 19. Acne involves increased sebum secretion, hyperkeratinization in the infrainfundibulum of the follicular duct, increased microbial colonization and inflammation (Strauss, J.S., *J. Dermatol*. *Treat*., 1:3-6 (1989)). Various methods for the treatment of acne and other sebaceous glands' inflammation have been proposed, ranging from special diets, prevention of contact of the skin by known acneignic agents (e.g., low grade cosmetics), use of endocrine preparations containing progesterone or estrogen, and others, most of which have not proved to be effective. Additionally, it has also been proposed to use antiseptic, antibacterial and wide-spectrum antibiotic compounds in both topical and systemic application.

All hitherto used anti-acne agents were effective in suppressing the development of microbial population, keratinization and comedo formation in the sebaceous glands. However, only few of the anti-acne agents hitherto used were effective in the reduction of the sebum excretion rate (Gollnick, H., *J. Dermatol. Treat* 1:S23-S28 (1990) and none of the agents was useful in affecting lipid biosynthesis in the pilosebaceous unit.

Isoprenoid groups such as cholesterol, squalene and cholesteryl-esters are synthesized via the mevalonate pathway (Goldstein, J.L, Brown, M.S., *Nature,* **34B,** 425 (1990), wherein the end-product is cholesterol. One of the key enzymes which regulate the production of mevalonate, the precursor of the above isoprenoid groups, is the 3-hydroxy-3-methylglutaryl coenzyme A (HMG-CoA) reductase. Inhibitors of this enzyme inhibit the synthesis of cholesterol and are thus used as antihypercholesterolemic medicaments for the treatment of arteriosclerosis, hyperlipemia and related diseases. An example of such an inhibitor is Lovastatin (Merck Index 5460, U.S. 4,231,938). Pharmaceutical compositions comprising this inhibitor of HMG-CoA reductase are given orally or parenterally to patients suffering from arteriosclerosis or hyperlipemia.

WO 92/00076 is directed to a pharmaceutical or cosmetic composition containing a combination of a retinoid and a cholesterol biosynthesis inhibitor sterol. This combination allows a synergic effect to be obtained, particularly in the treatment of epidermal keratinization disorders, epidermal proliferation disorders and /or sebaceous function disorders.

WO 87/04346 is directed to a pharmaceutical composition comprising an oxygenated cholesterol and a penetration- enhancing agent which is useful for topical application to the skin of a patient suffering from a proliferative skin disease.

EP 0599 203 A1 is directed to N,N-disubstituted arylcycloalkyl-amines, salts thereof, pharmaceutical compositions containing these compounds and the use thereof as well as methods for preparing these compounds.

EP 059 326 A1 is directed to arylidene-1-azacycloalkanes and arylallyl-1-azacycloalkanes, their suits, medicaments containing the same, use thereof and processes for their production.

US 5,075,327 relates to compounds which are also useful in lowering cholesterol, process of making the compounds and methods of resily the compounds to tread hyper proliferative skin diseases, such as psoriasis.

### SUMMARY OF THE INVENTION

In accordance with the invention it has surprisingly been found that acne can be treated by the use of a topically applied inhibitor of cholesterol synthesis, wherein the inhibitor of cholesterol synthesis is an inhibitor of statin type of the 3-hydroxy-3-methylgentaryl coenzyme A (HHG-CoA) reductase. In accordance with the invention use is thus made with an inhibitor of cholesterol synthesis to treat various skin disorders, wherein the inhibitor of cholesterol synthesis is an inhibitor of statin type of the 3-hydroxy-3-methylgentaryl coenzyme A (HHG-CoA) reductase.

In accordance with the present invention there is thus provided a composition for topical skin application comprising a carrier and, as an active ingredient, an effective amount of an inhibitor of cholesterol synthesis, wherein the inhibitor of cholesterol synthesis is an inhibitor of statin type of the 3-hydroxy-3-methylgentaryl coenzyme A (HHG-CoA) reductase.

The composition of the invention may be a pharmaceutical or cosmetic composition.

The pharmaceutical composition of the invention may be used for various indications including acne vulgaris, scalp dandruff and seborrhea.

The present invention further concerns the use of HMG-CoA reductase inhibitors of station type, for the preparation of topical pharmaceutical compositions for the treatment, alleviation or prevention of skin disorders.

Also provided by the invention is the use of a composition comprising a carrier and, as an active ingredient, an effective amount of an HMG-CoA reductase statin type for improvement of skin condition wherein the composition is to use topically applyied onto the skin. A particular application of the use is the treatment, alleviation or prevention of acne.

The term *"effective amount"* should be understood as meaning an amount of an active ingredient needed to achieve a desired therapeutic or pharmaceutical effect. For example, in a pharmaceutical composition of the invention an effective amount of an inhibitor of cholesterol synthesis is an amount which is sufficient, in the administration regimen of the pharmaceutical composition in the framework of treatment, to achieve an improvement in the skin's condition.

Inhibitors of cholesterol synthesis useful in accordance with the present invention are HMG -CoA reductase inhibitor agents which inhibit the production of the end product, i.e. cholesterol, or any of the intermediates of the various steps of the mevalonate pathway in which cholesterol is produced from the precursors acety CoA and acetoacetyl CoA.

In accordance with a preferred embodiment of the invention, the inhibitor of cholesterol synthesis is an agent which inhibits the HMG-CoA reductase, such as Lovastatin.

The concentration of the Lovastatin is preferably about 0.2 - 10% and most preferably about 2%.

The inhibitor of cholesterol synthesis, wherein the inhibitor of cholesterol synthesis is an inhibitor of statin type of the 3- hydroxy-3-methylgurtaryl coenzyme A (HHG-CoA) reductase may be applied to the skin with various other agents such as, antimicrobial agents, e.g. antibiotics, for the treatment or prevention of a secondary infection, a skin peeling agent, retin-A separately or together with resorcinol, etc.

The carrier of the composition of the present invention may be any pharmaceutically or cosmetically acceptable carrier such as, for example, ethanol, gel, liposome formulation, ointment, salve, etc.

### EXAMPLES:

### I. Preparation of the Composition

Lovastatin capsules (Mevacor™, Merck, U.S.A.) were ground and the active ingredient was separated from the excipient by extraction with ethanol 95% and filtration to yield a 2% solution of Lovastatin in ethanol.

### II. Clinical Trials

The efficacy of the above preparation was tested in two separate clinical trials.

### A. Trial I

Pharmaceutical compositions prepared as described above were topically applied twice daily for a period of 12 weeks, to the faces of two individuals suffering from acne vulgaris. The patients were required to discontinue all other topical and systemic anti-acne treatment 30 days prior to the beginning of the trial and discontinued all facial and cosmetic treatment seven days prior to the onset of treatment.

The acne condition was assessed by recording all acne lesions including inflamed acne lesions (papules and pustules) and non-inflamed acne lesions, (white and black comedos) prior to the beginning of treatment and 4, 8 and 12 weeks following the onset of treatment.

In both patients, improvement in all mentioned lesions was noticed and at the end of the 12 week treatment period the number of lesions decreased to less than half. No side effects were noticed save for a mild dryness of the skin, which is likely a result of the ethanol.

### B. Trial II

4 patients, 16-25 years of age, consisting of 2 males and 2 females, having mild to moderate acne were treated with the above preparation. All medications and cosmetics were stopped for 14 days, following which the patients were asked to apply the preparation twice daily for 8 weeks and to refrain from using all other forms of treatment and cosmetics during treatment. Prior to and after 4 and 8 weeks of treatment, the number of acne lesions (papules, pustules and white and black comedos) was recorded, and the results, shown in the following Table 1 demonstrated an improvement in all 4 patients evidenced by reduction of the number of all types of lesions:

**Table 1 Number of acne lesions before and during treatment**

| **Patient** | **Lesions** | **Before Treatment** | **After 1 month** | **After 2 months** |
|---|---|---|---|---|
| 1 | Pustules | 10 | 7 | 3 |
| | Papules | 11 | 3 | 2 |
| | White & blackheads | 18 | 10 | 7 |
| 2 | Pustules | 17 | 15 | 2 |
| | Papules | 17 | 15 | 10 |
| | White & blackheads | 18 | 15 | 6 |
| 3 | Pustules | 7 | 2 | - |
| | Papules | 12 | 7 | 4 |
| | White & blackheads | 22 | 14 | 7 |
| 4 | Pustules | 20 | 18 | 5 |
| | Papules | 16 | 9 | 5 |
| | White & blackheads | 15 | 10 | 5 |
| Average | Pustules | 13 | 10 | 2 |
| | Papules | 14 | 8 | 5 |
| | White & blackheads | 18 | 12 | 6 |

## Claims

1. Use as a sole active ingredient a of a 3-hydroxy-3-methylglutaryl coenzyme A (HMG-CoA) reductase inhitiator of statin type, for the preparation of a topical pharmaceutical composition for treating a skin disorder selected from the group consisting of acne, scalp dandruff or seborrhea,

2. The use of Claim 1, wherein said inhibitor is Lovastatin,

3. The use of Claim 2, wherein the concentration of Lovastatin in the composition is 0.2-10%.

4. The use of Claim 3, wherein the concentration of Lovastatin in the composition is about 2%.

5. The use of any one of Claims 1 to 4, wherein said skin disorder is acne.

## Patentansprüche

1. Verwendung, als einziger aktiver Bestandteil, eines 3-Hydroxy-3-methylglutaryl-Coenzym A (HMG-CoA) Reduktase-Inhibitors des Statintyps für die Herstellung eines lokalen Arzneimittels zur Behandlung von Hauterkrankungen ausgewählt aus Akne, Kopfschuppen oder Seborrhöe.

2. Verwendung nach Anspruch 1, wobei der Inhibitor Lovastatin ist.

3. Verwendung nach Anspruch 2, wobei die Konzentration des Lovastatins in der Zusammensetzung 0,2-10 % ist.

4. Verwendung nach Anspruch 3, wobei die Konzentration des Lovastatins in der Zusammensetzung etwa 2 % ist.

5. Verwendung nach einem der Ansprüche 1 bis 4, wobei die Hauterkrankung Akne ist.

## Revendications

1. Utilisation d'un inhibiteur de la 3-hydroxy-3-méthylglutaryl coenzyme A (HMG-CoA) réductase, du type statine, en tant que seul ingrédient actif, pour la préparation d'une composition pharmaceutique topique pour traiter une maladie de la peau choisie dans le groupe constitué de l'acné, des pellicules du cuir chevelu ou d'une séborrhée.

2. Utilisation selon la revendication 1, dans laquelle ledit inhibiteur est la Lovastatine.

3. Utilisation selon la revendication 2, dans laquelle la concentration de Lovastatine dans la composition est de 0,2-10%.

4. Utilisation selon la revendication 3, dans laquelle la concentration de Lovastatine dans la composition est d'environ 2%.

5. Utilisation selon l'une quelconque des revendications 1 à 4, dans laquelle ladite maladie de la peau est l'acné.
